# EUROPÄISCHE PATENTSCHRIFT

(11) **EP 1 719 500 B1**
(45) Veröffentlichungstag und Bekanntmachung des Hinweises auf die Patenterteilung: **30.06.2010**
(21) Anmeldenummer: 06113291.6
(22) Anmeldetag: 28.04.2006
(51) Int. Cl.: A61K 8/81, A61Q 5/06

(54) **Polymerkombination für kosmetische Zubereitungen**
Polymer mixture for cosmetic compositions.
Combinaison de polymères pour compositions cosmétiques.

(30) Priorität: 02.05.2005 DE 102005020706
(43) Veröffentlichungstag der Anmeldung: 08.11.2006
(73) Patentinhaber: Beiersdorf AG, 20253 Hamburg (DE)
(72) Erfinder: Franck, Kerstin, 22529, Hamburg (DE); Detert, Marion, 22455, Hamburg (DE); Saß, Viola, 25488, Holm (DE)

(56) Entgegenhaltungen:
- WO-A-99/02122
- US-B1- 6 482 394
- ANONYMOUS: "LuvimerÂ Pro 55 - Acrylates Terpolymer for Cosmetic compositions" IP.COM JOURNAL, IP.COM INC., WEST HENRIETTA, NY, US, 14. Januar 2005 (2005-01-14), XP013022837 ISSN: 1533-0001
- ROHM AND HAAS COMPANY: "Uses of Ethylenecarboxamide / Acrylamidomethylpropanesulfonate / Methacrylates polymer in personal care applications: Acudyne SCP styling conditioning polymer" RESEARCH DISCLOSURE, MASON PUBLICATIONS, HAMPSHIRE, GB, Bd. 478, Nr. 2, Februar 2004 (2004-02), XP007133356 ISSN: 0374-4353
- WANG M ET AL: "ACRYLATES/HYDROXYESTERS ACRYLATES COPOLYMER IN PERSONAL CARE APPLICATIONS: ACUDYNE DHR DURABLE HOLD RESIN" RESEARCH DISCLOSURE, MASON PUBLICATIONS, HAMPSHIRE, GB, Bd. 478, Nr. 6, Februar 2004 (2004-02), XP007133360 ISSN: 0374-4353

## Beschreibung

Die vorliegende Erfindung betrifft eine wässrige oder wässrig-alkoholische kosmetische Zubereitung zur Frisurgestaltung des menschlichen Kopfhaares enthaltend
a) ein anionisches Acrylatharz mit einer Molmasse zwischen 50 000 und 150 000 und einer Glasübergangstemperatur von 75 °C bis 105 °C in einer Konzentration von 0,01 bis 10 Gew.-% und
b) eine kationische Pflegesubstanz in einer Konzentration von 0,01 bis 5 Gew.-%,
jeweils bezogen auf das Gesamtgewicht der Zubereitung.

Der ganze menschliche Körper mit Ausnahme der Lippen, der Handinnenflächen und der Fußsohlen ist behaart, zum Großteil allerdings mit kaum sichtbaren Wollhärchen. Wegen der vielen Nervenden an der Haarwurzel reagieren Haare empfindlich auf äußere Einflüsse wie Wind oder Berührung und sind daher ein nicht zu unterschätzender Bestandteil des Tastsinns. Die wichtigste Funktion des menschlichen Kopfhaares dürfte allerdings heute darin bestehen, das Aussehen des Menschen in charakteristischer Weise mitzugestalten. Ähnlich wie die Haut erfüllt es eine soziale Funktion, da es über sein Erscheinungsbild erheblich zum Selbstwertgefühl des Individuums beiträgt.

Das Haar besteht aus dem frei aus der Haut herausragenden Haarschaft - dem keratinisierten (toten) Teil, der das eigentlich sichtbare Haar darstellt - und der in der Haut steckenden Haarwurzel - dem lebenden Teil, in dem das sichtbare Haar ständig neu gebildet wird. Der Haarschaft seinerseits ist aus drei Schichten aufgebaut: einem zentralen Teil - dem so genannten Haarmark (Medulla), welches allerdings beim Menschen zurückgebildet ist und oft gänzlich fehlt - ferner dem Mark (Cortex) und der äußeren, bis zu zehn Lagen starken Schuppenschicht (Cuticula), die das ganze Haar umhüllt.

Natürliches Haar hängt meist schlaff und ohne Volumen vom Kopf herab. Ein Ziel der Haarpflege ist es daher, dem Haar Fülle und Frisur zu geben. Zur temporären Verformung des Haares und Gestaltung (engl. Styling) vielseitiger Frisuren verwendet man Haarfestiger, meist in Form von Schaumfestigern oder Haarsprays. Beide haarkosmetischen Mittel sind in Ihrer Zusammensetzung ähnlich, unterscheiden sich aber in ihrer Aufgabe und Anwendung. Schaumfestiger (auch Schaumhaarfestiger genannt) werden in der Regel zur Frisurgestaltung im feuchten Haar verteilt, Haarsprays zur Fixierung auf die (trockene) fertig gestylte Frisur gesprüht. Neben den wegen ihrer leichten und angenehmen Anwendbarkeit zunehmend beliebten Schaumhaarfestigern und flüssigen Haarfestigern werden auch Haarfestigergele angeboten.

Die Rezepturen für derartige Produkte sind vielfältig. Die Bestandteile müssen jedoch in ethanolischem, wässrig-ethanolischem oder wässrigem Medium verträglich sein.

Üblicherweise bestehen diese Mittel zur Festigung der Haare (Haarfestiger) aus Lösungen von filmbildenden natürlichen oder synthetischen Polymeren oder Polymerkombinationen. Es handelt sich dabei um nichtionische, anionische, kationische oder amphotere Polymere. Diese hinterlassen auf dem Haar nach der Applikation einen transparenten farblosen Film. Es werden eine Reihe von Anforderungen an diese Filme gestellt: sie sollen klar, glänzend, feuchtigkeitsunempfindlich, festigend, lang anhaltend, flexibel, nicht klebend sein, den natürlichen Griff des Haares nicht beeinträchtigen und sich auch wieder leicht durch handelsübliche Haarwaschmittel auswaschen lassen. Schaumfestiger besitzen in der Gruppe der Stylingprodukte ein Sonderstellung. Nicht nur die Filmeigenschaften (Halt, Flexibilität...) stehen im Vordergrund, sondern auch die Pflegeleistung am Haar (Kämmbarkeit, gepflegter Griff) und die Schaumcharakteristik (feinblasig, cremig, stabil) haben einen beträchtlichen Einfluss auf die Produktperformance.

Die Auswahl des geeigneten Polymers (nichtionisch, anionisch, kationisch oder amphoter) ist von entscheidender Bedeutung für die Eigenschaften des Endproduktes.

Kationische Filmbildner weisen vor allem pflegende Eigenschaften auf: das Haar lässt sich gut kämmen und fühlt sich geschmeidig und gepflegt an. Insbesondere für Schaumfestiger hat die Kämmbarkeit und der Griff der nassen und trockenen Haare eine entscheidende Bedeutung. Als entscheidender Nachteil dieser Rohstoffe ist ihre geringe Festigungsleistung zu nennen.

Anionische oder amphotere Filmbildner werden in Haarfestigern eingesetzt, um eine gute und lang anhaltende Festigung der Frisur zu erreichen. Ein entscheidender Nachteil dieser Rohstoffe sind jedoch ihre mangelhaften pflegenden Eigenschaften: das Haar lässt sich schlecht kämmen und fühlt sich stumpf, rau und wenig gepflegt an. Nach dem Trocknen sind die Filme dann relativ hart, spröde und unflexibel. Nachteilig für den Verbraucher sind die taktilen Eigenschaften des Filmes sowie die Bildung von Rückständen beim Kämmen (Filmplaken). Diese rieseln auf Schultern oder Kleidung und lassen den Anwender ungepflegt erscheinen. Bei Schaumfestigern kommt darüber hinaus ein weiterer Nachteil vieler anionischer und amphoterer Polymere zum Tragen: sie verleihen dem Schaum eine unzureichende, unkosmetische Schaumcharakteristik. Der Schaum ist grobblasig, weich, instabil und wenig cremig. Zur Verbesserung der Schaumeigenschaften ist der Zusatz von Schaum stabilsierenden Additiven, wie z.B. Emulgatoren üblich. Besonders bevorzugt ist der Einsatz von Laureth-3.

Die Verwendung von Polymermischungen zur Produktoptimierung von haarkosmetischen Zubereitungen ist dem Fachmann an sich bekannt.

Eine Verbesserung in Bezug auf Griff und Kämmbarkeit wird bei Styling-Formulierungen auf Basis anionischer Polymere typischerweise durch die Kombination mit kationischen Polymeren, kationischen Tensiden oder Silikonen (beispielsweise cyclische Polydimethylsiloxane (Cyclomethicone), Silikontenside (Polyethermodifizierte Siloxane) vom Typ Dimethicone) erreicht. Bei derartigen Zubereitungen besteht jedoch immer die Gefahr einer Ausfällung der Polymere durch die Bildung von Polymersalzen aus anionischen und kationischen Polymeren. Außerdem führt der Einsatz dieser kationischen Polymere oder kationischen Tenside meistens zu einer deutliche Abnahme der Festigungsleistung und des Langzeithaltes der Frisur.

Es war daher die Aufgabe der vorliegenden Erfindung, die Mängel des Standes der Technik zu beseitigen und Zubereitungen (insbesondere Mittel zur Frisurgestaltung) zu entwickeln, die zu einer guten Kämmbarkeit des Haares führen. Das Haar sollte sich nach der Anwendung nicht mehr stumpf anfühlen, sondern einen glatten, gut gepflegten Eindruck hinterlassen. Die Bildung von Filmplaken sollte unterdrückt sein. Festigungsleistung und Langzeithalt sollten gegenüber dem Stand der Technik erhöht sein. Darüber hinaus sollte sich das Produkt zu einem feinblasigen, cremigen, stabilen Schaum aufschäumen lassen.

Überraschend gelöst werden die Aufgaben durch eine wässrige oder wässrig-alkoholische kosmetische Zubereitung zur Frisurgestaltung des menschlichen Kopfhaares enthaltend
a) ein anionisches Acrylatharz mit einer Molmasse zwischen 50 000 und 150 000 und einer Glasübergangstemperatur von 75 °C bis 105 °C in einer Konzentration von 0,01 bis 10 Gew.-% und
b) mindestens eine kationische Pflegesubstanz in einer Konzentration von 0,01 bis 5 Gew.-%, jeweils bezogen auf das Gesamtgewicht der Zubereitung, **dadurch gekennzeichnet, dass** als anionisches Acrylatharz ein lineares Tetrapolymer mit zufälliger Verteilung der Monomere eingesetzt wird, das aus den Monomeren Methacrylsäure, Hydroxyethylmethacrylat, Methylmethacrylat und Butylacrylat gebildet wird.

### Zwar kennt der Stand der Technik die Dokumente

"Acrylates terpolymer Luvimer Pro55 for cosmetic compositions", IP.com Journal XP13022837 vom 14.1.2005, ISSN 1533-0001,
"Uses of Ethylenecarboxamide/Acrylamidomethylpropynsulfonate/Methacrylates Polymer in Personal Care Applications; Acudyne SCP Styling Conditioning Polymer", Research disclosure mason publications Bd. 478, Nr.2, XP007133356 vom Februar 2004, ISSN 0374-4353, "Acrylates/Hydroxyesters Acrylates Copolymer in Personal Care Applications: Acudyne DHR Durable Hold Resin", Research disclosure mason publications Bd. 478, Nr.6, XP007133360 vom Februar 2004, ISSN 0374-4353,
US 6,482,394 und WO 9/02122, doch konnten diese Schriften nicht den Weg zur vorliegenden Erfindung weisen.

Die erfindungsgemäßen Zubereitungen führen zu einer deutlichen Verbesserung der Pflegeleistung unter gleichzeitiger Erhöhung der Festigungsleistung bei ihrer Anwendung als Haarfestiger.

Der Langzeithalt der Frisur gemessen im "Curl-Retention-Test" bei 90 % r.F. ist deutlich erhöht.

Liegen die erfindungsgemäßen Zubereitungen in Form eines Schaumfestigers vor, so weist die Zubereitung einen besonders cremigen, feinblasigen Schaum auf, der sich überraschend leicht und gleichmäßig im Haar verteilen lässt.

Das Haar lässt sich nach der Anwendung besonders leicht kämmen und weist einen gesunden, seidigen Glanz auf. Die Bildung von aus dem Haar herab rieselnden Filmplaken ist deutlich reduziert.

Es ist erfindungsgemäß bevorzugt, wenn das anionische Acrylatharz mit einer Molmasse zwischen 50 000 und 150 000 und einer Glasübergangstemperatur von 75 °C bis 105 °C in einer Konzentration von 5 bis 7 Gew.-%, bezogen auf das Gesamtgewicht der Zubereitung in dieser enthalten ist.

Es ist erfindungsgemäß bevorzugt, wenn die kationische Pflegesubstanz in einer Konzentration von 2 bis 4 Gew.-%, bezogen auf das Gesamtgewicht der Zubereitung, in dieser enthalten ist.

Es ist erfindungsgemäß vorteilhaft, wenn die erfindungsgemäße Zubereitung in Form eines Schaums vorliegt.

Dabei ist es erfindungsgemäß vorteilhaft, wenn die erfindungsgemäße Zubereitung 0,01 bis 15 Gewichts-% und bevorzugt 8 bis 11 Gewichts-%, jeweils bezogen auf das Gesamtgewicht der Zubereitung, Treibmittel enthält.

Erfindungsgemäß vorteilhafte Treibmittel sind Propan, Isobutan und n-Butan sowie deren Mischungen. Erfindungsgemäß bevorzugt wird als Treibmittel eine Mischung aus Propan und Butan eingesetzt wird.

Natürlich weiß der Fachmann, dass es an sich nichttoxische Treibgase gibt, die grundsätzlich für die Verwirklichung der vorliegenden Erfindung in Form von Aerosolpräparaten geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Es ist erfindungsgemäß bevorzugt, wenn das erfindungsgemäße anioische Acrylatharz die folgende Struktur aufweist: 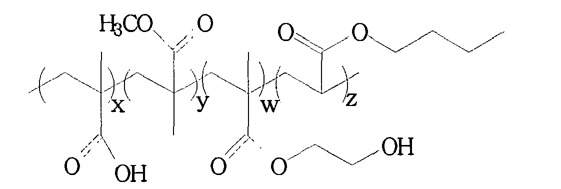 das heißt, wenn es sich um ein lineares Tetrapolymer mit zufälliger Verteilung der Monomere handelt, wobei als Monomere Methacrylsäure, Hydroxyethylmethacrylat, Methylmethacrylat und Butylacrylat eingesetzt werden.

Dieses ist unter der INCI Acrylates/Hydroxyesteracrylates Copolymer registriert und kann beispielsweise unter dem Handelsnamen Acudyne 180 bei der Firma Rohm & Haas erworben werden.

Als kationische Pflegesubstanzen können sowohl kationische Polymere und/oder kationische Tenside eingesetzt werden.

Als erfindungsgemäß vorteilhafte kationische Polymere können Polyquaternium-4, Polyquaternium-6, Polyquaternium-7, Polyquaternium-10, Polyquaternium-11, Polyquaternium-16, Polyquaternium-22, Polyquaternium-44, Polyquaternium-46, Polyquaternium-55 und/oder quaternisierte Guarderivate eingesetzt werden.

Erfindungsgemäß besonders bevorzugt ist es, wenn als kationische Polymere Polyquaternium-4, Polyquaternium-16 oder Polyquaternium-44 eingesetzt werden. Beispielsweise können die folgenden kationischen Polyquaternium-Typen eingesetzt werden: Celquat H-100 oder Celquat L-200 (Fa. National Starch); INCI: Polyquaternium-4, Luviquat Excellence, Luviquat FC-370, Luviquat FC-550 oder Luviquat Style (Fa.: BASF); INCI: Polyquaternium-16, Luviquat Care oder Luviquat Ultra Care (Fa. BASF); INCI: Polyquaternium-44.

Als erfindungsgemäß vorteilhafte kationische Tenside können Verbindungen aus der Gruppe der quaternären Ammoniumverbindungen, insbesondere Benzyltrialkylammoniumsalze oder Alkyltrimethylammoniumsalze, beispielsweise Cetyltrimethylammoniumchlorid oder -bromid oder -methosulfat, Alkyldimethylhydroxyethylammoniumchloride oder -bromide oder - methosulfat, Dialkyldimethylammonium-chloride oder -bromide oder -methosulfat, Esterquats wie z.B. Diacylethyl Hydroxyethylmethylammoniumchlorid oder -bromid oder - methosulfat, Alkylamidethyltrimethylammoniumethersulfate, Hydroxyethyl Cetyldimonium Phosphate, Alkylpyridiniumsalze, beispielsweise Lauryl- oder Cetylpyrimidiniumchlorid, Imidazolinderivate und Verbindungen mit kationischem Charakter wie Aminoxide, beispielsweise Alkyldimethylaminoxide oder Alkylaminoethyl-dimethylaminoxide eingesetzt werden.

Erfindungsgemäß besonders bevorzugt ist es, wenn als kationisches Tensid Hydroxyethylcetyldimoniumphosphat eingesetzt wird.
Beispielsweise kann das folgende kationische Hydroxyethylcetyldimoniumphosphat eingesetzt werden: Luviquat Mono CP (Fa. BASF); INCI: Hydroxyethyl Cetyldimonium Phosphate.

Die erfindungsgemäße Zubereitung enthält erfindungsgemäß vorteilhaft weitere Komponenten.

So sind erfindungsgemäß vorteilhafte Ausführungsformen **dadurch gekennzeichnet, dass** die Zubereitung von 0,1 bis 2,0 Gew.-% Laureth-3 enthält, wobei sich die Gewichtsangabe auf das Gesamtgewicht der Zubereitung bezieht.

Ferner sind erfindungsgemäß vorteilhafte Ausführungsformen **dadurch gekennzeichnet, dass** die Zubereitung von 0,1 bis 2,0 Gew.-% Aminomethylpropanol enthält, wobei sich die Gewichtsangabe auf das Gesamtgewicht der Zubereitung bezieht.

Erfindungsgemäß vorteilhafte Ausführungsformen der vorliegenden Erfindung sind auch **dadurch gekennzeichnet, dass** die Zubereitung von 0,01 bis 0,5 Gew.-% Panthenol und/oder Niacinamid enthält, wobei sich die Gewichtsangabe auf das Gesamtgewicht der Zubereitung bezieht.

Nicht zuletzt sind erfindungsgemäß vorteilhafte Ausführungsformen der vorliegenden Erfindung **dadurch gekennzeichnet, dass** die Zubereitung Parabene in einer Gesamtkonzentration von bis zu 0,5 Gewichts-%, bezogen auf das Gesamtgewicht der Zubereitung bezieht.

Die kosmetischen und dermatologischen Zubereitungen können erfindungsgemäß kosmetische Hilfsstoffe enthalten, wie sie üblicherweise in solchen Zubereitungen verwendet werden, z.B. Konservierungsmittel, Antioxidanien, Bakterizide, Parfüme, Emulgatoren, weichmachende, anfeuchtende und/oder feuchthaltende Substanzen, Fette, Öle, Wachse, Farbstoffe, Pigmente, die eine färbende Wirkung haben, Verdickungsmittel oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate. Auch die Einarbeitung von UV-Lichtschutzfiltern ist erfindungsgemäß vorteilhaft. Die aufgeführte Liste an Zusatzstoffen soll selbstverständlich nicht limitierend sein.

Erfindungsgemäß bevorzugt ist die Verwendung der kosmetische Zubereitung als Haarfestiger. Erfindungsgemäß besonders bevorzugt ist die Verwendung der erfindungsgemäßen Zubereitung als Schaumfestiger für die Frisurgestaltung.
Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### Beispiele

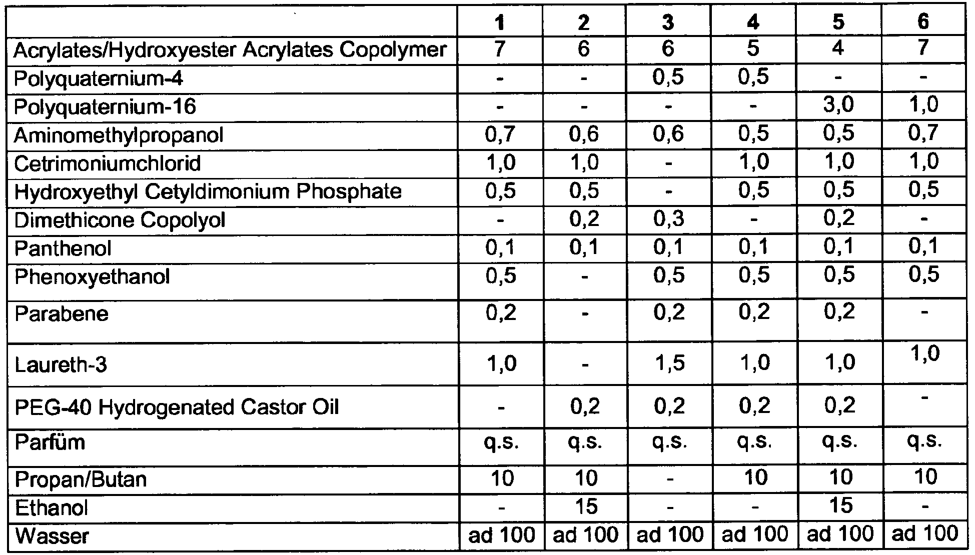

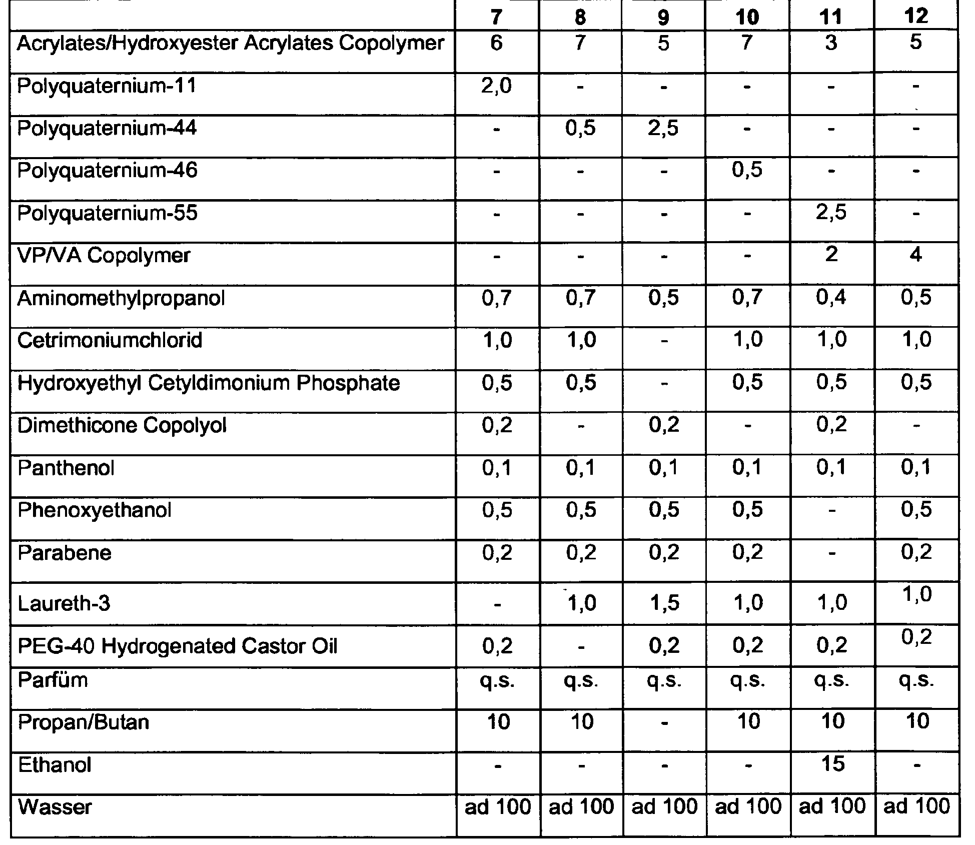

## Patentansprüche

1. Wässrige oder wässrig-alkoholische kosmetische Zubereitung zur Frisurgestaltung des menschlichen Kopfhaares enthaltend
a) ein anionisches Acrylatharz mit einer Molmasse zwischen 50 000 und 150 000 und einer Glasübergangstemperatur von 75 °C bis 105 °C in einer Konzentration von 0,01 bis 10 Gew.-% und
b) mindestens eine kationische Pflegesubstanz in einer Konzentration von 0,01 bis 5 Gew.-%,
jeweils bezogen auf das Gesamtgewicht der Zubereitung, **dadurch gekennzeichnet, dass** als anionisches Acrylatharz ein lineares Tetrapolymer mit zufälliger Verteilung der Monomere eingesetzt wird, das aus den Monomeren Methacrylsäure, Hydroxyethylmethacrylat, Methylmethacrylat und Butylacrylat gebildet wird.

2. Zubereitung nach Anspruch 1, **dadurch gekennzeichnet, dass** die Zubereitung in Form eines Schaums vorliegt.

3. Zubereitung nach einem der Ansprüche 1 oder 2, **dadurch gekennzeichnet, dass** die Zubereitung 0,01 bis 15 Gewichts-% eines Treibmittels enthält.

4. Zubereitung nach einem der vorhergehenden Ansprüche, **dadurch gekennzeichnet, dass** als Treibmittel eine Mischung aus Propan und Butan eingesetzt wird.

5. Zubereitung nach einem der vorhergehenden Ansprüche, **dadurch gekennzeichnet, dass** als kationische Pflegesubstanz das Tensid Hydroxyethylcetyldimoniumphosphat eingesetzt wird.

6. Zubereitung nach einem der vorhergehenden Ansprüche, **dadurch gekennzeichnet, dass** als kationische Pflegesubstanz Polyquaternium-4 oder Polyquaternium-16 oder Polyquaternium-44 eingesetzt werden.

7. Zubereitung nach einem der vorhergehenden Ansprüche, **dadurch gekennzeichnet, dass** die Zubereitung von 0,1 bis 2 Gew.-% Laureth-3 enthält, wobei sich die Gewichtsangabe auf das Gesamtgewicht der Zubereitung bezieht.

8. Zubereitung nach einem der vorhergehenden Ansprüche, **dadurch gekennzeichnet, dass** die Zubereitung von 0,1 bis 2 Gew.-% Aminomethylpropanol enthält, wobei sich die Gewichtsangabe auf das Gesamtgewicht der Zubereitung bezieht.

9. Zubereitung nach einem der vorhergehenden Ansprüche, **dadurch gekennzeichnet, dass** die Zubereitung von 0,01 bis 0,5 Gew.-%Panthenol und/oder Niacinamid enthält, wobei sich die Gewichtsangabe auf das Gesamtgewicht der Zubereitung bezieht.

## Claims

1. Aqueous or aqueous-alcoholic cosmetic preparation for styling human head hair comprising
a) an anionic acrylate resin with a molar mass between 50 000 and 150 000 and a glass transition temperature from 75°C to 105°C in a concentration from 0.01 to 10% by weight and
b) at least one cationic care substance in a concentration from 0.01 to 5% by weight,
in each case based on the total weight of the preparation, **characterized in that** the anionic acrylate resin used is a linear tetrapolymer with random distribution of the monomers which is formed from the monomers methacrylic acid, hydroxyethyl methacrylate, methyl methacrylate and butyl acrylate.

2. Preparation according to Claim 1, **characterized in that** the preparation is in the form of a foam.

3. Preparation according to either of Claims 1 and 2, **characterized in that** the preparation comprises 0.01 to 15% by weight of a propellant.

4. Preparation according to one of the preceding claims, **characterized in that** the propellant used is a mixture of propane and butane.

5. Preparation according to one of the preceding claims, **characterized in that** the cationic care substance used is the surfactant hydroxyethyl-cetyldimonium phosphate.

6. Preparation according to one of the preceding claims, **characterized in that** the cationic care substance used is polyquaternium-4 or polyquaternium-16 or polyquaternium-44.

7. Preparation according to one of the preceding claims, **characterized in that** the preparation comprises from 0.1 to 2% by weight of laureth-3, where the stated weight refers to the total weight of the preparation.

8. Preparation according to one of the preceding claims, **characterized in that** the preparation comprises from 0.1 to 2% by weight of aminomethylpropanol, where the stated weight refers to the total weight of the preparation.

9. Preparation according to one of the preceding claims, **characterized in that** the preparation comprises from 0.01 to 0.5% by weight of panthenol and/or niacinamide, where the stated weight refers to the total weight of the preparation.

## Revendications

1. Préparation aqueuse ou aqueuse-alcoolique pour le coiffage des cheveux humains, contenant
a) une résine acrylate anionique ayant une masse moléculaire comprise entre 50 000 et 150 000 et une température de transition vitreuse de 75 °C à 105 °C à une concentration de 0,01 à 10 % en poids et
b) au moins une substance de soin cationique à une concentration de 0,01 à 5 % en poids,
chaque fois par rapport au poids total de la préparation, **caractérisée en ce qu'**on utilise comme résine acrylate anionique un tétrapolymère linéaire à distribution aléatoire des monomères, qui est formé à partir des monomères acide méthacrylique, méthacrylate d'hydroxyéthyle, méthacrylate de méthyle et acrylate de butyle.

2. Préparation selon la revendication 1, **caractérisée en ce que** la préparation se trouve sous forme d'une mousse.

3. Préparation selon la revendication 1 ou 2, **caractérisée en ce que** la préparation contient de 0,01 à 15 % en poids d'un agent d'expansion.

4. Préparation selon l'une quelconque des revendications précédentes, **caractérisée en ce qu'**on utilise comme agent d'expansion un mélange de propane et butane.

5. Préparation selon l'une quelconque des revendications précédentes, **caractérisée en ce qu'**on utilise comme substance de soin cationique le tensioactif phosphate d'hydroxyéthylcétyldimonium.

6. Préparation selon l'une quelconque des revendications précédentes, **caractérisée en ce qu'**on utilise comme substance de soin cationique le Polyquaternium-4 ou le Polyquaternium-16 ou le Polyquaternium-44.

7. Préparation selon l'une quelconque des revendications précédentes, **caractérisée en ce que** la préparation contient de 0,1 à 2 % en poids de Laureth-3, la donnée pondérale se rapportant au poids total de la préparation.

8. Préparation selon l'une quelconque des revendications précédentes, **caractérisée en ce que** la préparation contient de 0,1 à 2 % en poids d'aminométhylpropanol, la donnée pondérale se rapportant au poids total de la préparation.

9. Préparation selon l'une quelconque des revendications précédentes, **caractérisée en ce que** la préparation contient de 0,01 à 0,5 % en poids de panthénol et/ou de niacinamide, la donnée pondérale se rapportant au poids total de la préparation.
